# EUROPEAN PATENT APPLICATION

(11) **EP 2 832 362 A1**
(43) Date of publication of application: **04.02.2015**
(21) Application number: 13178951.3
(22) Date of filing: 01.08.2013
(51) Int. Cl.: A61K 38/17, A61K 35/54, A61K 35/34, A61P 9/10

(54) **glycosylated protein of an extra-cellular matrix for use in a method of treating an ischemic heart of a human or animal subject in need thereof**

(71) Applicant: Fraunhofer Gesellschaft zur Förderung der angewandten Forschung e.V., 80686 München (DE)
(72) Inventor: Layland, Shannon, 71034 Böblingen (DE); Schenke-Layland, Katja, 71034 Böblingen (DE); Holeiter, Monika, 70569 Stuttgart (DE)
(74) Representative: Obermeier, Andreas

(57) **Abstract**

The present invention relates to a glycosylated protein of an extra-cellular matrix, preferably of a mammalian extra-cellular matrix for use in a method of treating an ischemic heart of a human or animal subject in need thereof, a pharmaceutical composition comprising said glycosylated protein and a method for the in vitro production of differentiated cardiomyocytes.

## Description

The present invention relates to a glycosylated protein of an extracellular matrix, preferably of a mammalian extra-cellular matrix for use in a method of treating an ischemic heart of a human or animal subject in need thereof, a pharmaceutical composition comprising said glycosylated protein and a method for the in vitro production of differentiated cardiomyocytes.

Ischemia is a restriction in blood supply which can result in damage or dysfunction of a tissue. Ischemia of a heart muscle due to a cardiovascular disease, in particular due to a myocardial infarction, is a major problem that has to be tackled by modern medicine. Heart failure is the number one cause of death in industrialized countries. Myocardial infarction typically results in fibrotic scar formation and permanently impaired cardiac function because, after a massive cell loss due to ischemia, the myocardial tissue lacks intrinsic regenerative capability. In subjects, which survived a myocardial infarction cardiac collagen accumulation takes place and stiffened coliagenrich scar tissue is formed, which could result in a hypertrophy of the remaining myocardium as well as pathological deformation of the left ventricle and finally in heart failure.

EP 1 730 265 A2 discloses a composition comprising a biodegradable gel-based matrix, at least one active agent and stem cells being able to differentiate into cardiac tissue.

EP 1 856 246 A2 discloses decellularized tissue-derived extra-cellular matrices and methods of generating and using the same. The methods of generating a decellularized matrix include the step of (a) subjecting the tissue to washes and a hypertonic buffer, (b) subjecting the tissue to an enzymatic proteolytic digestion with an enzyme, such as trypsin, and (c) removing all cellular components from the tissue using a detergent solution which includes Triton-X-100 and ammonium hydroxide.

EP 2 349 292 A2 discloses a composition comprising decellularized extra-cellular matrix derived from cardiac tissue.

Seif-Naraghi et al (Sci Transl Med, 2013, 5 (173), 173) disclose safety and efficacy of an injectable extra-cellular matrix hydrogel for treating myocardial infarction.

Sreejit et al. (Stem Cell Reviews and Reports, 2013, 9 (2), 158 to 171) disclose natural extra-cellular matrix as biomaterial for scaffold based cardiac regeneration using adult bone marrow derived stem cells.

Higuchi et al. (J Biosci Bioeng, 2013, 115(3), 320 to 5) disclose that heart extra-cellular matrix supports cardiomyocyte differentiation of mouse embryonic stem cells.

Hinderer et al. (Biomaterials, 2012, 33(21), 5259 to 66) disclose engineering of fibrillar decorin matrices for a tissue-engineered trachea.

Thus, various approaches have been provided to develop strategies for improving the extent and quality of regeneration of damage, in particular of ischemic heart tissue. However, none of the disclosed methods provide a satisfactory result. Accordingly, there is still an unmet demand for improved means and method to regenerate ischemic heart tissue.

The technical problem underlying the present invention is, in particular, to overcome the above identified disadvantages, preferably to provide a component, preferably a pharmaceutical composition, which improves the differentiation of pluripotent stem cells, cardiac stem cells and/or cardiac progenitor cells, into differentiated myocardiocytes, that means a higher number of cells should differentiate towards the cardiac lineage than without said component, preferably said pharmaceutical composition. In particular, by the use of the component and/or pharmaceutical composition to be provided the heart tissue of an animal or human subject that has suffered or is suffering ischemia should be superiorly regenerated compared to a heart tissue of an animal or human subject not be treated with said component and/or pharmaceutical composition.

Said technical problem has been solved by the teaching of the independent claims.

In context with the present invention, the glycosylated protein according to the present invention and/or the pharmaceutical composition according to the present invention is/are preferably a glycosylated protein and/or a pharmaceutical composition of one or more preferred embodiments thereof as disclosed therein.

In context with the present invention the term "cell" is understood in its broadest sense in the art and refers to a living body which is a structural unit of tissue of a multicellular organism, is surrounded by a membrane structure which isolates it from the outside, has the capability of self-replicating, and has genetic information and a mechanism for expressing it. Cells used herein may be naturally-occurring cells or artificially modified cells, for instance fusion cells and genetically modified cells.

In the context of the present invention, the term "stem cell" refers to a cell capable of giving rise to at least one type of a more specialized cell. A stem cell has the ability to self-renew, that is to go through numerous cycles of cell division while maintaining the undifferentiated state, and has the capacity to differentiate into specialized cell types. Usually, stem cells can regenerate an injured tissue. Stem cells herein are preferably embryonic stem cells, induced pluripotent stem cells, or tissue stem cells, also called tissue-specific stem cell or somatic stem cell.

"Pluripotent stem cells" in the context of the present invention are cells that can differentiate into cells derived from any of the three germ layers, for example, direct descendants of totipotent stem cells or induced pluripotent stem cells. Thus, the term "pluripotent stem cells" includes also induced pluripotent stem cells.

"Induced pluripotent stem cells" commonly abbreviated as iPS cells or iPSCs, refer to a type of pluripotent stem cell artificially prepared from a non-pluripotent cell, typically an adult somatic cell, or terminally differentiated cell, such as fibroblast, a hematopoietic cell, a myocyte, a neuron, an epidermal cell, or the like, by inserting certain genes, referred to as reprogramming factors.

"Embryonic stem cells" in the context of the present invention are cells derived from mammalian embryos or blastocysts, which are self-renewing and have the ability to yield many or all of the cell types present in a mature animal or human. Embryonic stem cells are pluripotent stem cells. Under defined conditions embryonic stem cells are capable of propagating themselves indefinitely. The pluripotency distinguishes embryonic stem cells from "adult stem cells" found in adults. While embryonic stem cells can generate all cell types in the body, adult stem cells are multipotent and can produce only a limited number of cell types.

"Adult stem cells", also known as somatic stem cells, are undifferentiated cells, found throughout the body after development, that multiply by cell division to replenish dying cells and regenerated damaged tissues. They can be found in juvenile as well as adult animals and human bodies. Adult stem cells are able to divide or self-renew indefinitely and generate all the cell types of an organ from which they originate, potentially regenerating the entire organ from a few cells. Adult stem cells possess the properties of self-renewal and multipotency. "Multipotency" means to have the ability to generate progenitors of several distinct cell types.

Unlike embryonic stem cells, "cardiac stem cells" have a limited differentiation potential. Cardiac stem cells are tissue-specific stem progenitor cells, preferably tissue-specific progenitor cells within an adult mammalian heart.

"Progenitor cells" are cells with the capability of differentiating into several different cell types, but not any cell type. These cells are known as oligopotent or multipotent. The most important difference between stem cells and progenitor cells is that stem cells can replicate indefinitely, whereas progenitor cells can divide only a limited number of times. Progenitor cells are in a later stage of cell differentiation in comparison to stem cells. Thus, in the process of differentiating cells progenitor cells are present between stem cells and fully differentiated cells.

In the context of the present invention "cardiomyocytes", also called cardiac muscle cells or myocardiocytes, are muscle cells that make up the cardiac muscle. Each cardiomyocyte contains myofibrils, which are long chains of sarcomeres, the contractile units of muscle cells. Cardiomyocytes show striations similar to those on skeletal muscle cells, but unlike multinucleated skeletal cells, they contain only one nucleus. The term "cardiomyocytes" includes cardiomyocyte-like cells that exhibit some but not all characteristics or mature cardiomyocytes, as well as mature and fully functional cardiomyocytes, preferably fully functional cardiomyocytes, which have all characteristics of cardiomyocytes as determined by morphology, marker expression, in vitro and in vivo functional assays.

The term "embryoid bodies" is understood as aggregates of differentiated and undifferentiated cells which can be preferaby produced as described in Example 1, below, or by adding pieces of monolayered aggregated differentiated and undifferentiated cells to a substrate which they cannot adhere to.

As used herein, the term "carrier" refers to a diluent, adjuvant, excipient, or vehicle with which a compound is administered. Pharmaceutically acceptable carriers are preferably sterile liquids, such as water, oils and hydrogels. Water or aqueous solution saline solutions and aqueous dextrose and glycerol solutions are preferably employed as carriers, particularly for injectable solutions. Most preferably hydrogels are employed as carriers, particularly for injectable media.

In context with the present invention the term "in therapeutically effective amounts" means that at least 10%, preferably at least 20%, preferably at least 50%, preferably at least 60%, preferably at least 70%, preferably at least 80%, preferably at least 90%, preferably at least 99%, preferably 100% the differentiable, preferably undifferentiated cells, preferably selected from the group consisting of pluripotent stem cells, cardiac stem cells, cardiac progenitor cells and combinations thereof, are differentiated to cardiomyocytes. Preferably the at least one glycosylated protein is administrated to the human or animal subject in need thereof in therapeutically effective amounts, to achieve at least 1.1 times, at least 1.2 times, preferably at least 1.3 times, preferably at least 1.4 times, preferably at least 1.5 times, preferably at least 2 times, preferably at least 5 times, preferably at least 10 times as many differentiable, preferably undifferentiated cells selected from the group consisting of embryonic stem cells, cardiac stem cells, cardiac progenitor cells and combinations thereof, are differentiated to cardiomyocytes, compared to differentiable, preferably undifferentiated cells which are differentiated under the same conditions without the addition of the glycosylated protein, especially in a pharmaceutical composition.

A "biomolecule" which "encodes" a particular protein, is in the context of the present invention a nucleic acid molecule which is transcribed and optionally also translated into a gene product, for instance a polypeptide, in vitro or in vivo when placed under the control of appropriate regulatory sequences. The coding region may be present in a cDNA, genomic DNA, or RNA form. When present in a DNA form, the nucleic acid molecule may be single-stranded, that is the sense strand, or double-stranded. The boundaries of a coding region are determined by a start codon at the 5' (amino) terminus and a translation stop codon at the 3' (carboxy) terminus.

The term "recombinant DNA molecule" is understood in the context of the present invention as a DNA molecule that is comprised of segments of DNA joined together by means of molecular biological techniques.

The term "recombinantly produced protein" is understood in the context of the present invention as protein that is expressed from a recombinant DNA molecule. Recombinant DNA (rDNA) molecules are DNA sequences that are comprised of segments of DNA joined together by means of molecular biological techniques.

In context with the present invention the term "in vitro" is understood as an artificial environment and as processes or reactions that occur within an artificial environment. In vitro environments can preferably comprise test tubes and cell culture.

The term "extra-cellular matrix" means a collagen-rich substance that is found in between cells in animal or human tissue and serves as a structural element in tissues. It typically comprises a complex mixture of polysaccharides and proteins secreted by cells. The extra-cellular matrix used can be isolated and treated in a variety of ways. Extra-cellular matrix material (ECM) is preferably isolated from small intestine submucosa, stomach submucosa, urinary bladder submucosa, tissue mucosa, dura mater, liver basement membrane, pericardium or other tissues. Following isolation and treatment, it is commonly referred to as extra-cellular matrix or ECM material.

The term "pharmaceutical composition" is understood as formulation which provides some therapeutic and/or beneficial effect, preferably produces a localized or systemic effect or effects in animals or humans, preferably in warm blooded mammals, preferably in humans.

In context with the present invention the term "subject in need thereof" is understood as any animal or human subject that has suffered or is suffering from ischemia, preferably from one or more of the following: myocardial infarction, chronic heart failure, ischemic heart disease, coronary artery disease, diabetic heart disease, thrombotic stroke or embolic stroke. The animal or human subject is preferably a warm blooded mammal, preferably a human.

The term "administration" means that a pharmaceutical composition or an active agent formulation is provided to a treatment site, for instance a damaged tissue, through any method appropriate to deliver the functional agent or formulation or composition to the treatment site. Delivery methods are preferably direct injection, percutaneous delivery and topical application at the treatment site.

In the context of the present invention the terms "treatment" and "treating" mean obtaining a desired pharmaceutical and/or physiological effect. Preferably, the effect is therapeutic in terms of partially or completely curing ischemia. The term "treatment" as used herein covers any treatment of heart tissue suffering from ischemia and/or heart defects and/or heart dysfunction caused by ischemia in a human or animal, preferably a mammal, particularly a vertebrate and more preferably a human, and includes regenerating and/or repairing at least partially the heart or heart tissue dysfunction.

The term "ischemia", also called cardiac ischemia, is understood in the context with the present invention as a condition that may occur in the heart tissue that is suffering a lack of oxygen supply and/or supply with metabolites which occurs when there is an imbalance between oxygen supply and demand, due to inadequate perfusion, that leads to insufficient oxygen in the heart tissue. Also medical interventions such as the interruption of the blood flow, for instance during bypass surgery may lead to ischemia. Cardiac ischemia includes a broad variety of conditions, from silent ischemia to stable or unstable angina to myocardial infarction (AMI or "heart attack").

In accordance with the present invention decorin is one of the glycosylated proteins of extra-cellular matrix, preferably of the mammalian extra-cellular matrix used in the method of treating the ischemic heart. "Decorin" is a proteoglycan having on average 90 to 140 kilodaltons (kD) in molecular weight. It belongs to the small leucine-rich proteoglycan (SLRP) family and consists of a protein core containing leucine repeats with a glycosaminoglycan (GAG) chain consisting of either chondroitin sulfate (CS) or dermatan sulfate (DS). Decorin is a small cellular or pericellular matrix proteoglycan which is a component of connective tissue, binds to type I collagen fibrils, and plays a role in matrix assembly. Decorin appears to influence fibrillogenesis, and also interacts with fibronectin, thrombospondin, the complement component C1q and epidermal growth factor receptor (EGFR).

In accordance with the present invention nidogen-1 is one of the glycosylated proteins of the extra-cellular matrix, preferably of the mammalian extra-cellular matrix used in the method of treating the ischemic heart. "Nidogen-1", also called entactin, is a sulfated multidomain glycoprotein component of basement membranes, alongside other components such as collagen type IV, proteoglycans, such as heparan sulfate and glycosaminoglycans, laminin and fibronectin. Nidogen-1 binds calcium ions and this calcium-binding activity of nidogen-1 may play a role in the matrix assembly process. Its major function appears to be the assembly of the basement membrane.

The technical problem of the present invention is particularly solved by a glycosylated protein of an extra-cellular matrix, preferably of a mammalian extra-cellular matrix for the use in a method of treating an ischemic heart of a human or animal subject in need thereof, wherein the glycosylated protein of the extra-cellular matrix, preferably the mammalian extra-cellular matrix is selected from the group consisting of decorin, nidogen-1 and a combination thereof.

It has been surprisingly found that the extra-cellular matrix proteins decorin and nidogen-1 play a significant role in the cardiovascular differentiation of differentiable, preferably undifferentiated cells selected from the group consisting of embryonic stem cells, cardiac stem cells, cardiac progenitor cells and combinations thereof. Both proteins can be used for achieving the differentiation of pluripotent stem cells and/or progenitor cells into the cardiovascular lineage. Thus, by using the extra-cellular matrix proteins decorin and nidogen-1 conditions and an environment for the differentiation of the stem cells and/or progenitor cells are provided in the ischemic heart, which leads to differentiated cardiomyocytes.

In a preferred embodiment of the present invention the glycosylated proteins of the extra-cellular matrix, preferably of the mammalian extra-cellular matrix are recombinantly produced proteins, preferably recombinantly produced human proteins. By recombinant production the glycosylated protein of the extra-cellular matrix, preferably of the mammalian extra-cellular matrix can be produced in a reproducible and controlled way and can be administrated to the subject in need thereof in a defined concentration.

The recombinant production of the human protein decorin and/or nidogen-1 takes preferably place in host cell lines, preferably Chinese hamster ovary host cell lines (CHO-host cell lines). CHO-host cell lines have been approved by the Food and Drug Administration (FDA) for the recombinant production of protein therapeutics due to their high safety level. For clinical studies the production of the recombinantly produced, preferably human, proteins can further be adjusted to good manufacturing practice (GMP) conditions.

The glycosylated proteins and/or the pharmaceutical composition according to the present invention are preferably contamination-free, preferably free of viruses, prions and other not yet known risks, which can be transferred to the subject in need thereof, in particular free of viruses and prions.

Especially if recombinantly produced human proteins are used, they will not be recognised as being foreign by the human immune system, which would result in an adverse immune reaction. The same applies to recombinantly produced proteins of a specific animal, when administered to the same specific animal.

In a preferred embodiment of the present invention, the pharmaceutical composition and/or the glycosylated proteins will not only be administrated, preferably injected, into the damaged heart tissue, but also to the adjacent regions thereof.

The technical problem of the present invention is also particularly solved by a pharmaceutical composition for use in a method of treating an ischemic heart of a human or animal subject in need thereof, comprising at least one glycosylated protein of an extra-cellular matrix, preferably a mammalian extra-cellular matrix selected from the group consisting of decorin, nidogen-1 and a combination thereof.

In a preferred embodiment of the present invention both nidogen-1 and decorin are used, preferably in the pharmaceutical composition, in a method of treating an ischemic heart.

In a preferred embodiment of the present invention the glycosylated protein of an extra-cellular matrix, preferably of a mammalian extra-cellular matrix selected from the group consisting of decorin, nidogen-1 and a combination thereof is used and/or administered in therapeutically effective amounts.

The present invention relates in a preferred embodiment to a pharmaceutical composition according to the present invention, comprising further at least one pharmaceutically acceptable carrier.

The present invention relates in a preferred embodiment to a glycosylated protein according to the present invention or pharmaceutical composition according to the present invention, wherein the treating of the ischemic heart comprises regenerating the heart tissue.

The present invention relates in a preferred embodiment to a pharmaceutical composition according to the present invention, wherein the at least one protein of the extra-cellular matrix, preferably of the mammalian extra-cellular matrix is comprised in a two or three dimensional scaffold. The two dimensional scaffold is preferably a mesh. The three dimensional scaffold is preferably a hydrogel, an electro-spun, bioprinted or biosprayed three-dimensional scaffold or a native tissue scaffold.

The present invention relates in a preferred embodiment to a pharmaceutical composition according to the present invention, wherein the at least one protein of the extra-cellular matrix, preferably of the mammalian extra-cellular matrix is comprised in a hydrogel.

The present invention relates in a preferred embodiment to a pharmaceutical composition according to the present invention, wherein the at least one protein of the extra-cellular matrix, preferably of the mammalian extra-cellular matrix is comprised in an electro-spun three-dimensional scaffold.

The present invention relates in a preferred embodiment to a pharmaceutical composition according to the present invention, wherein the at least one protein of the extra-cellular matrix, preferably of the mammalian extra-cellular matrix is present together with differentiable, preferably undifferentiated cells selected from the group consisting of embryonic stem cells, cardiac stem cells, cardiac progenitor cells and combinations thereof.

The technical problem of the present invention is particularly solved by the use of a glycosylated protein of an extra-cellular matrix, preferably of a mammalian extra-cellular matrix selected from the group consisting of decorin, nidogen-1 and a combination thereof for the treatment of an ischemic heart of a human or animal subject in need thereof.

Thus, the present invention also relates to the use of the aboveidentified glycosylated protein of an extra-cellular matrix, preferably of a mammalian extra-cellular matrix for the preparation of a pharmaceutical composition for the treatment of ischemic heart of a human or animal subject in need thereof.

The technical problem of the present invention is also particularly solved by the use of a pharmaceutical composition for treating an ischemic heart of a human or animal subject in need thereof, comprising at least one glycosylated protein of an extra-cellular matrix, preferably of a mammalian extra-cellular matrix selected from the group consisting of decorin, nidogen-1 and a combination thereof.

The present invention relates in a preferred embodiment to a use according to the present invention, wherein the treating of the ischemic heart comprises regenerating the heart tissue.

The present invention relates in a preferred embodiment to a use according to the present invention, wherein the at least one protein of the extra-cellular matrix, preferably of the mammalian extra-cellular matrix is administrated to the subject in need thereof in a two or three dimensional scaffold. The two dimensional scaffold is preferably a mesh. The three dimensional scaffold is preferably a hydrogel, an electro-spun, bioprinted or biosprayed three-dimensional scaffold or a native tissue scaffold, preferably a hydrogel and/or an electro-spun three-dimensional scaffold.

The present invention relates in a preferred embodiment to a use according to the present invention, wherein the at least one protein of the extra-cellular matrix, preferably of the mammalian extra-cellular matrix is administrated to the subject in need thereof together with differentiable, preferably undifferentiated cells selected from the group consisting of embryonic stem cells, cardiac stem cells, cardiac progenitor cells and combinations thereof.

In the preferred embodiment the at least one protein of the extra-cellular matrix, preferably of the mammalian extra-cellular matrix is provided in injectable form for injection in the ischemic heart of the subject in need thereof.

The present invention relates in a preferred embodiment to a use according to the present invention, wherein the at least one protein of the extra-cellular matrix, preferably of the mammalian extra-cellular matrix is administrated by injection in the ischemic heart of the subject in need thereof.

The present invention relates in a preferred embodiment to a use according to the present invention, wherein the at least one protein of the extra-cellular matrix, preferably of the mammalian extra-cellular matrix is a recombinantly produced protein.

The technical problem of the present invention is also particularly solved by a method for the in vitro production of differentiated cardiomyocytes, wherein differentiable, preferably undifferentiated cells selected from the group consisting of embryonic stem cells, cardiac stem cells, cardiac progenitor cells and combinations thereof are cultivated with at least one biomolecule being or encoding a glycosylated protein of an extra-cellular matrix, preferably of a mammalian extra-cellular matrix selected from the group consisting of decorin, nidogen-1 and a combination thereof, so as to obtain differentiated cardiomyocytes. The biomolecule encoding the glycosylated protein of the extra-cellular matrix, preferably of the mammalian extra-cellular matrix is preferably present in host cells translating said biomolecule into the glycosylated proteins decorin and/or nidogen-1.

In a preferred embodiment of the present invention, the cardiac stem cells are cardiac adult stem cells.

The technical problem of the present invention is also particularly solved by a method for treatment of an ischemic heart of a human or animal subject in need thereof by administering a glycosylated protein of an extra-cellular matrix, preferably of a mammalian extra-cellular matrix selected from the group consisting of decorin, nidogen-1 and a combination thereof.

The technical problem of the present invention is also particularly solved by a method for treatment of an ischemic heart of a human or animal subject in need thereof by administering a pharmaceutical composition comprising at least one glycosylated protein of an extra-cellular matrix, preferably of a mammalian extra-cellular matrix selected from the group consisting of decorin, nidogen-1 and a combination thereof.

Preferably, the treatment of the ischemic heart comprises regenerating the heart tissue.

The at least one, preferably recombinantly produced, protein of the extra-cellular matrix, preferably of the mammalian extra-cellular matrix is preferably administrated to the subject in need thereof together with or in a two or three dimensional scaffold, preferably by injection into the ischemic heart of the subject in need thereof. The two dimensional scaffold is preferably a mesh. The three dimensional scaffold is preferably a hydrogel, an electro-spun, bioprinted or biosprayed three-dimensional scaffold or a native tissue scaffold, preferably a hydrogel or an electro-spun three-dimensional scaffold.

More preferably the at least one protein of the extra-cellular matrix, preferably of the mammalian extra-cellular matrix is administrated to the subject in need thereof together with differentiable, preferably undifferentiated cells selected from the group consisting of embryonic stem cells, cardiac stem cells, cardiac progenitor cells and combinations thereof.

Most preferably the at least one protein of the extra-cellular matrix, preferably of the mammalian extra-cellular matrix is comprised together with differentiable, preferably undifferentiated cells, selected from the group consisting of embryonic stem cells, cardiac stem cells, cardiac progenitor cells and combinations thereof, in a two or three dimensional scaffold, preferably a mesh, a hydrogel, an electro-spun, bioprinted or biosprayed three-dimensional scaffold or a native tissue scaffold, preferably a hydrogel or an electro-spun three-dimensional scaffold.

The technical problem of the present invention is also particularly solved by a kit comprising (a) a glycosylated protein of an extracellular matrix, preferably of a mammalian extra-cellular matrix selected from the group consisting of decorin, nidogen-1 and a combination thereof, and (b) cells selected from the group consisting of embryonic stem cells, cardiac stem cells, cardiac progenitor cells and combinations thereof. Preferably, the kit further comprises an extra-cellular matrix, preferably a mammalian extra-cellular matrix. Preferably, the kit comprising (a) and (b) further comprises a two or three dimensional scaffold.

In the method of treating an ischemic heart of an animal or human subject in need thereof according to the present invention, wherein the heart that has suffered or is suffering from ischemia, the glycosylated protein of the extra-cellular matrix, preferably of the mammalian extra-cellular matrix, namely decorin and/or nidogen-1, is used for differentiating stem cells and/or progenitor cells, especially selected from the group consisting of embryonic stem cells, cardiac stem cells, cardiac progenitor cells and combinations thereof, into differentiated, preferably fully differentiated, cardiomyocytes.

In a preferred embodiment, the differentiated, preferably undifferentiated cells, preferably selected from the group consisting of embryonic stem cells, cardiac stem cells, cardiac progenitor cells and combinations thereof, preferably cardiac stem cells and/or cardiac progenitor cells are administrated to the human or animal subject in need thereof in form of agglomerates, preferably in a suspension or in a two or three dimensional scaffold. These agglomerates are called "embryoid bodies" in case of embryonic stem cells.

In a preferred embodiment of the present invention, the at least one glycosylated protein is administrated to the human or animal subject need thereof during or after suffering from ischemia. The term "after suffering" means preferably that the at least one glycosylated protein, namely decorin and/or nidogen-1, is administered 1 hour to 15 years, preferably 1 hour to 6 months, preferably 2 to 6 weeks after the beginning of the ischemia of the heart in a human or an animal subject.

In a preferred embodiment of the present invention, the differentiable, preferably undifferentiated cells, preferably selected from the group consisting of embryonic stem cells, cardiac stem cells, cardiac progenitor cells and combinations thereof, are administrated while being embedded in a two or three dimensional scaffold comprising the glycosylated protein selected from the group consisting of nidogen-1, decorin and a combination thereof.

In a preferred embodiment of the present invention, the glycosylated protein and/or the pharmaceutical composition is administrated into the subject in need thereof by injection directly into the peripheral circulation, heart muscle, left ventricle, right ventricle, coronary artery, cerebro-spinal fluid, neural tissue, ischemic tissue or postischemic tissue, in particular into the left ventricle, right ventricle, coronary artery, heart muscle tissue, ischemic tissue or postischemic tissue.

Further preferred embodiments are the subject-matter of the subclaims.

The present invention is illustrated by the figures and examples, below.

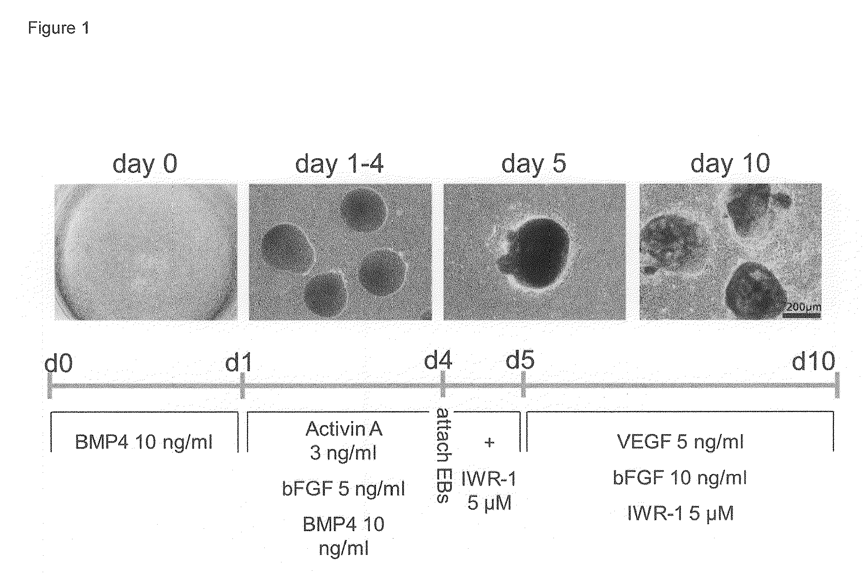

Figure 1 shows a modified cardiac differentiation protocol for spin embryoid bodies from human embryonic stem cells line H9 (the abbreviations used are explained in Examples 1 and 2, below).

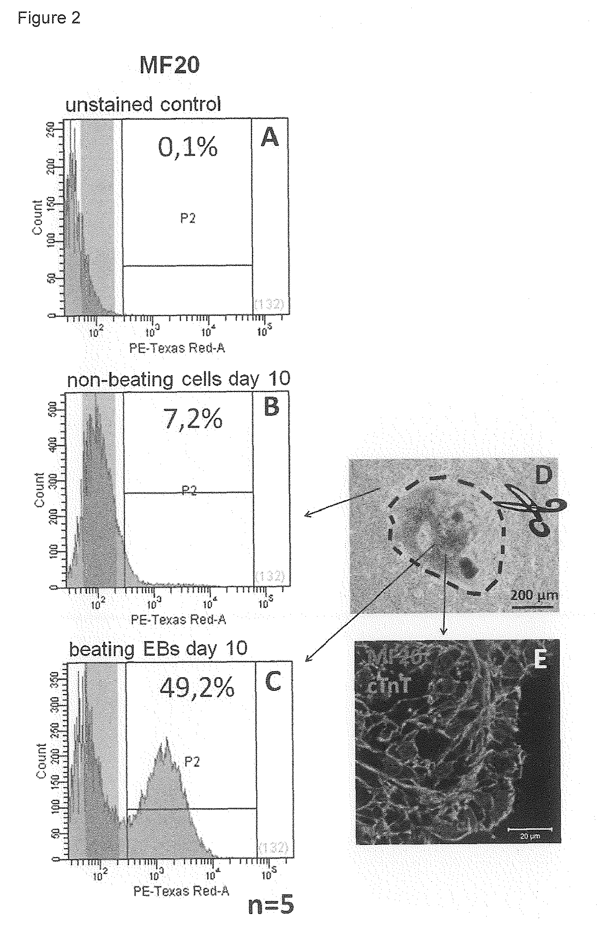

Figure 2 shows intracellular flow-cytometric analysis for sacromeric myosin (MF20 antibody), n = 5; Figure 2A: unstained control; Figure 2B: non-beating outgrown cells on day 10; Figure 2C: beating embryoid bodies on day 10; Figure 2D: samples for (B), (C), and (E) were dissect from the plate; Figure 2E: immunofluorescence image of the beating embryoid body on day 10 stained with sacromeric myosin (MF20) and cardiac troponin T. PE-Texas Red-A is is a conjugate of Texas Red, also called sulforhodamine 101 acid chloride, with R-phycoerythrin.

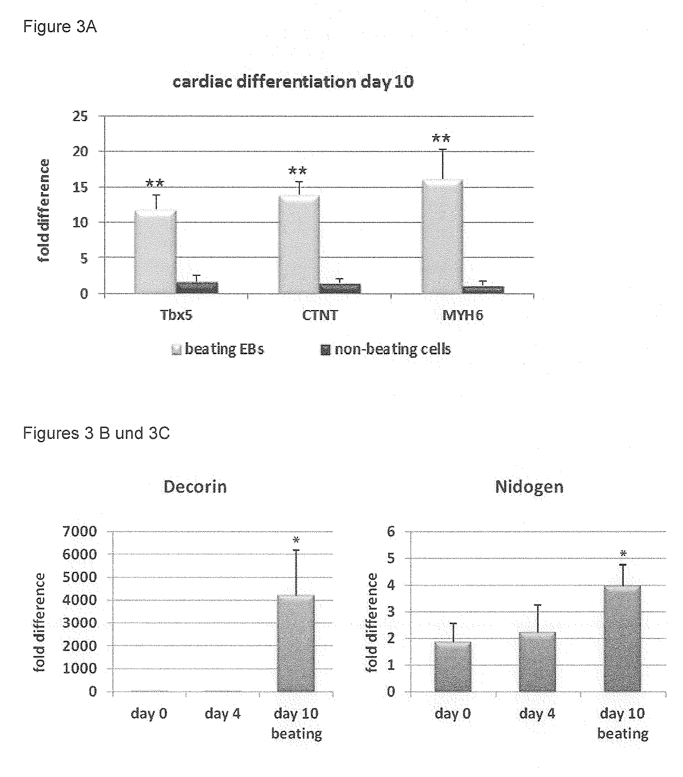

Figure 3 shows the result of the real time PCR-analysis; (A): of beating embryoid bodies and non-beating cells at day 10 of cardiac differentiation with focus on the description factor Tbx5, cardiac troponin T and cardiac muscle myosin MYH6; (B): gene expression of decorin, and (C): nidogen-1 during cardiac differentiation.

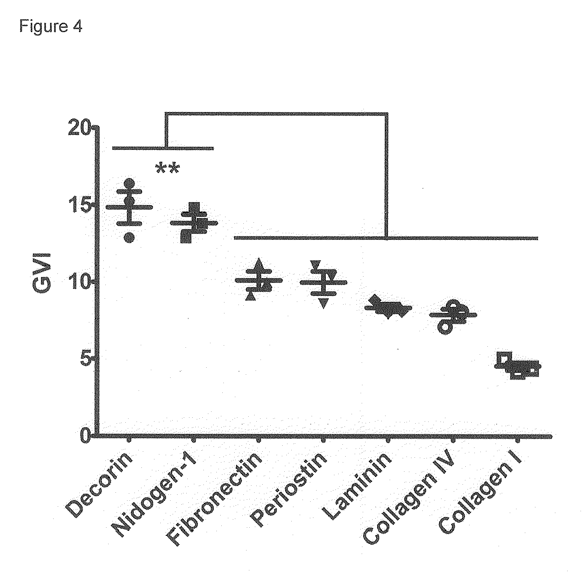

Figure 4 shows the comparison of the grey value intensities (GVI) of immunofluorescence images of beating embryoid bodies at day 10 of cardiac differentiation with focus on the extra-cellular matrix proteins decorin, nidogen-1, fibronectin, periostin, collagen I, collagen IV and laminin.

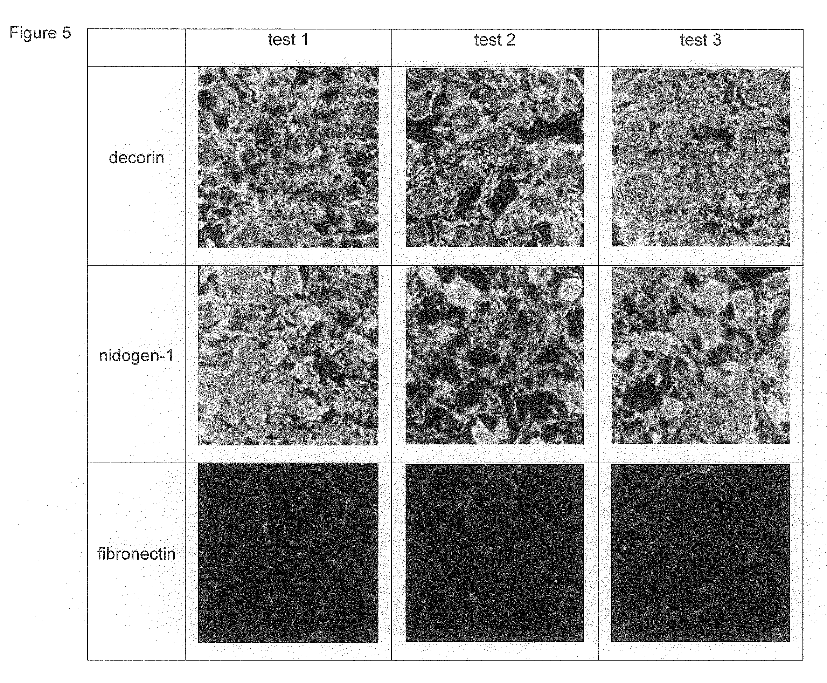

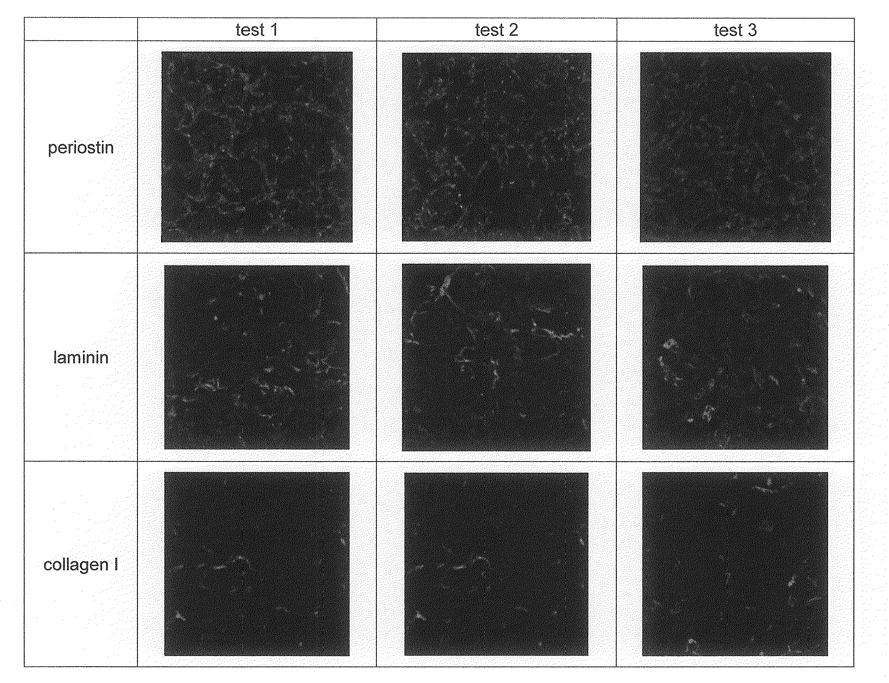

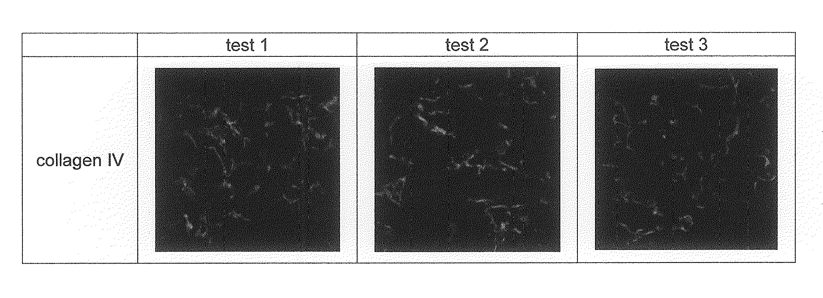

Figure 5 shows immunofluorescence images of beating embryoid bodies at day 10 of cardiac differentiation. The green stained (grey in figure 5) extra-cellular matrix proteins decorin, nidogen-1, fibronectin, periostin, laminin, collagen I and collagen IV were imaged with one of the three channels of a immunofluorescence microscope. The differentiated cardiomyocytes and the sacromeric myosin also present in the sample has not been made visible. The green stained (grey in
figure 5) extra-cellular matrix proteins have been found close to the differentiated cardiomyocytes.

The abbreviation "EBs" used in figures 2 and 3 means embryoid bodies.

### Examples:

### 1. Generation of embryoid bodies

hESCs (H9) (Thomson, J.A., et al., Embryonic stem cell lines derived from human blastocysts. Science, 1998. 282(5391): p. 1145 to 1147) were cultured according to current protocols (WiCell Research Institute, Inc.) on MEF (mouse embryonic fibroblast) cells (Millopore (#PMEF-CFL)) (see also figure 1). For single cell suspension, the H9 cell colonies were washed with PBS (phosphate buffered saline), incubated with TrypLE Select (Invitrogen, life technologies) 5 min at 37°C, resuspended with a 1 ml pipette and passed through a cell strainer (40 µm, BD Falcon). The cell suspension was mixed with PBS in the ratio 1:1 and centrifuged (5 min, 1000 rpm). The cell pellet was resuspended in mTeSR1 culture medium (STEMCELL Technologies, Inc., Vancouver, Canada) with 10 ng/ml BMP4 (bone morphogenetic protein 4) and 10 ng/ml Rho-associated kinase (ROCK) inhibitor Y-27632 (Sigma, St. Louis, MO) to make a 600000 cells/ml suspension. 30000 cells (50 µl of the cell suspension) were dispensed per well in a 96-well plate with conical wells (96-Well Microplates, non-treated, conical bottom, Fisher Scientific). The cell suspension in the 96-well plate was centrifuged at 1000 rpm for 5 min and the plate was incubated overnight in a cell culture incubator.

### 2. Modified cardiac differentiation protocol

For cardiac differentiation, we used a modification of the differentiation protocols described in Yang (Human cardiovascular progenitor cells develop from a KDR+ embryonic-stem-cell-derived population. Nature, 2008. 453(7194): p. 524 to 528) and Willems et al. (Smallmolecule inhibitors of the Wnt pathway potently promote cardiomyocytes from human embryonic stem cell-derived mesoderm. Circ Res, 2011. 109(4): p. 360 to 364). On day 1 of differentiation, after the ovornight incubation, EBs (embryoid bodies) formed due to cell aggregation and were carefully transferred to ultra-low attachment 6-well plates (Corning incorporated, New York, Product #3471) with 1 ml pipettes. One 6-well can hold up to 200 EBs in 3 ml of StemPro 34 (Life technologies, Carlsbad, California, U.S.) + 3 ng/ml Activin A + 5 ng/ml bFGF (basic fibroblast growth factor) + 10 ng/ml BMP4 (stage 1 media). A change of the same media is required on day 2 or 3 of differentiation. The floating EBs were transferred to a 0.1% Gelatin coated 6-well on day 4 with 2 ml of stage 1 media + 5 µM IWR-1 (4-(1,3,3a,4,7,7a-Hexahydro-1,3-dioxo-4,7-methano-2H-isoindol-2-yl)-N-8-quinolinyl-benzamide; as replacement of 150 ng/ml DKK-1). The EBs were incubated without moving the plate until the next day. On day 5, most of the EBs had attached to the dish and cells outgrew them. From day 5 to day 10 the attached EBs were differentiated in stage 2 media that consisted of StemPro 34 + 5 ng/ml VEGF (vascular endothelial growth factor) + 10 ng/ml bFGF + 5 pM IWR-1. A change of the same media was required on day 8 of differentiation. The attached EBs started beating on day 7 or 8 and were harvested for further analysis on day 10.

### 3. Analysis

To identify the differentiation status, flow-cytometric analysis were performed for sacromeric myosin (MF20 antibody) and immunofluorescence staining for cardiac troponin T (cTnT) and sacromeric myosin. Additionally, real-time PCR was utilized to monitor the expression of the transcription factor TbX5, cTnT and the alpha heavy chain of the cardiac muscle myosin (MYH6). The expression of the cardiac specific extra-cellular matrix during the differentiation process was analysed by real-time PCR and immunofluorescence.

### 4. Results

By employing the modified cell culture protocol, a high number of cells differentiated towards the cardiac lineage as identified by flow cytometric analysis (figure 2) and real-time PCR analysis (figure 3a) as well as immunofluorescence staining (figure 2e). It has been observed a robust and reproducible generation of spontaneously beating embryoid bodies (85% +/- 17.6 %). In addition, real-time PCR revealed a stage-specific and culture condition specific expression of extra-cellular matrix proteins with a significant increase in decorin and nidogen-1 expression during cardiac differentiation (figures 3b and 3c). Semi-quantitative analysis of immunofluorescence images (see figure 5) of beating embryoid bodies at day 10 of differentiation revealed are significantly higher amount of decorin and nidogen-1 as well as fibronectin compared to collagen IV and laminin in the beating embryoid bodies (figure 4 and table 1).

Table 1 shows the normalized grey value intensities of the images of figure 5.

**Table 1**

| protein | decorin | nidogen-1 | fibronectin | periostin | laminin | collagen IV | collagen I |
|---|---|---|---|---|---|---|---|
| test 1 | 16,405 | 14,831 | 9,136 | 11,042 | 8,017 | 8,048 | 4,398 |
| test 2 | 12,874 | 12,881 | 9,982 | 10,334 | 8,095 | 8,383 | 4,206 |
| test 3 | 15,240 | 13,846 | 11,219 | 8,573 | 8,817 | 7,097 | 4,970 |

## Claims

1. A glycosylated protein of an extra-cellular matrix for use in a method of treating an ischemic heart of a human or animal subject in need thereof, wherein the glycosylated protein of the extra-cellular matrix is selected from the group consisting of decorin, nidogen-1 and a combination thereof.

2. A pharmaceutical composition for use in a method of treating an ischemic heart of a human or animal subject in need thereof, comprising at least one glycosylated protein of an extra-cellular matrix selected from the group consisting of decorin, nidogen-1 and a combination thereof.

3. The pharmaceutical composition according to claim 2, comprising further at least one pharmaceutically acceptable carrier.

4. The glycosylated protein or pharmaceutical composition according to any one of the preceding claims, wherein the treating of the ischemic heart comprises regenerating the heart tissue.

5. The pharmaceutical composition according to any one of claims 2 to 4, wherein the at least one protein of the extra-cellular matrix is comprised in a hydrogel.

6. The pharmaceutical composition according to any one of claims 2 to 5, wherein the at least one protein of the extra-cellular matrix is comprised in an electro-spun three-dimensional scaffold.

7. The pharmaceutical composition according to any one of claims 2 to 6, wherein the at least one protein of the extra-cellular matrix is present together with differentiable cells selected from the group consisting of embryonic stem cells, cardiac stem cells, cardiac progenitor cells and combinations thereof.

8. The glycosylated protein or the pharmaceutical composition according to any one of claims 1 to 7, wherein the at least one protein of the extra-cellular matrix is provided in injectable form for injection in the ischemic heart of the subject in need thereof.

9. The glycosylated protein or the pharmaceutical composition according to any one of claims 1 to 8, wherein the at least one protein of the extra-cellular matrix is a recombinantly produced protein.

10. Method for the in vitro production of differentiated cardiomyocytes, wherein differentiable cells selected from the group consisting of embryonic stem cells, cardiac stem cells, cardiac progenitor cells and combinations thereof are cultivated with at least one biomolecule being or encoding a glycosylated protein of an extra-cellular matrix selected from the group consisting of decorin, nidogen-1 and a combination thereof, as to obtain differentiated cardiomyocytes.
